# EUROPEAN PATENT APPLICATION

(11) **EP 4 008 337 A1**
(43) Date of publication of application: **08.06.2022**
(21) Application number: 20839663.0
(22) Date of filing: 09.07.2020
(51) Int. Cl.: A61K 38/17, A61P 25/04, C07K 14/46

(54) **SYNERGISTIC EFFECT OF COBRA NEUROTOXIN POLYPEPTIDE ON INHIBITING OPIOID HYPERALGESIA AND TOLERANCE AND ALLEVIATING PAIN ASSOCIATED THEREWITH**

(30) Priority: 14.07.2019 CN 201910653374
(71) Applicant: Qi, Zhankai, Shanghai 200065 (CN)
(72) Inventor: QI, Hyatt, Shanghai 200065 (CN); QI, Zhankai, Shanghai 200065 (CN)
(74) Representative: Sun, Yiming
(86) International application number: PCT/CN2020/000149
(87) International publication number: WO 2021/008100

(57) **Abstract**

Opioid induced tolerance and hyperalgesia are two closely related yet different symptoms. The former analgesic tolerance refers to the decline in analgesic effects of an opioid, whilst the latter refers to the patient's abnormal pain response to non-injurious stimuli or a highly sensitive pain response to the same injurious stimuli caused by continuous applications of opioid receptor agonists. Analgesic tolerance and hyperalgesia, although two different adverse reactions induced by opioid receptor agonists, both impede the long-term clinical use of opioids. Consequently, patients under the long-term prescription of such drugs are left to face severe side effects, thus a major unmet clinical need.

Elapidae neurotoxin, not only it inhibits opioid induced hyperalgesia and tolerance, but also it can produce synergistic analgesic effect with an opioid for the treatment of pain, hence it decreases the severe side effect of opioid such as morphine, and synergizes the anti-nociceptive effect of opioid.

## Description

### FIELD OF THE INVENTION

This disclosure relates to compositions comprising elapidae neurotoxins and its new application in clinics, particularly in inhibiting opioids induced side effect and in synergizing opioids analgesic effect in treatment of pain.

### BACKGROUND OF THE INVENTION

Opium is a substance extracted from poppy plants which binds to the opioid receptors of the human body itself and provides pain relief or analgesia to patients with acute or chronic pain. Morphine is an example of an opioid analgesia. Short-term use of opioid compositions is considered as a safe pain relief method. However, when they are frequently administered and/or overused, a series of problems arise which include hyperalgesia, tolerance build-up, drug dependency, constipation, respiratory Inhibition, and fatal overdose. According to the CDC, in 2015, over 33,000 people died in US due to opioid-related overdoses.

Opioid receptor agonists such as morphine are the most widely used potent analgesic drug in clinics. The medicine can effectively relieve the pain of patients, especially post-operative and advanced cancer pain. However, the use of opioids such as morphine often leads to tolerance build-up and hyperalgesia in patients which can occur in as little as a week. And once tolerance and hyperalgesia develops, the patient requires increasing dosages to maintain the drug's initial effectiveness. In other words, the body needs a greater dose of medication to achieve the same analgesic effect as it initially provided; larger doses lead to greater hyperalgesia, the faster build-up of tolerance, constipation, addiction, and respiratory suppression. Morphine induced hyperalgesia and tolerance could be built-up regardless method of administration, dosage and administration time interval. Therefore, to achieve both the clinical efficacy and the acceptance of treatment, opioid induced hyperalgesia and tolerance are urgent problems in need of a real solution.

Opioid induced analgesic tolerance and hyperalgesia are two closely related yet different symptoms. The former refers to the significant decline in analgesic effects of an opioid, whilst the latter refers to the patient's abnormal pain response to non-injurious stimuli or a highly sensitive pain response to the same injurious stimuli caused by continuous or incorrect applications of opioid receptor agonists such as morphine. Analgesic tolerance and hyperalgesia, although two different adverse events induced by opioid receptor agonists, both impede the long-term clinical use of opioids. Consequently, patients under the long-term prescription of such compositions are left to face severe side effects, thus a major unmet clinical need.

Presently, treatments for opioids induced tolerance and hyperalgesia mostly remain in experimental stages, drug treatment includes dimethylaminocycline, pentoxifylline, statins, resveratrol, etc. Although the combination of opioids and other compositions might become an effective strategy for enhancing the analgesic effect of opioids, and to reduce the opioid dose, however, there is no singular drug with proven efficacy that could produce synergistic effect, and at same time, address analgesic tolerance and hyperalgesia. Thus clinically, there is a demand for a product that can fully satisfy the unmet needs of patients.

The part of elapidae neurotoxins that have postsynaptic blocking actions are widely referred to as a-neurotoxins.[1,2] they interfere with cholinergic transmission at various post-synaptic sites by binding to nicotinic acetylcholine receptors (nAChRs) reversely in the peripheral and central nervous systems. Nicotinic acetylcholine receptors (nAChR) are involved in the protection and neurotransmission of sensation, cognition, pain, [3] therefore, once blocked, the effects of cognition, pain, neuron protection and neurotransmission will be affected in different degrees.

### DETAILED DESCRIPTION OF THE INVENTION

The analgesic effect of elapidae neurotoxins have been previously reported, however, elapidae neurotoxin 's ability to inhibit or control opioid induced hyperalgesia and tolerance has never been disclosed before. Elapidae neurotoxins have no dependence on opioid system, and no hyperalgesia or tolerance were observed during analgesic process. When combined with opioids, such as morphine for example, the combination can at same time inhibit hyperalgesia, tolerance, and produce a synergistic analgesic effect and prolong opioid's effective time, which will allow low dose morphine to achieve same or even better analgesic effect than high dose morphine, thus can overcome the side effect of morphine for long term use in clinics.

In addition, due to the synergistic effect and the ability to prolong the analgesic effect of morphine, the combination can reduce the dose of morphine and increase the time interval of morphine's analgesic effect in clinics. These unique properties are also first proved in our discovery, thereby the elapidae neurotoxin, as an morphine replacement analgesia, due to these unique properties in controlling morphine's side effect , is expected be developed into an analgesic agent with synergy with morphine.

The primary purpose of the invention is to provide a composition of matter using elapidae neurotoxin to inhibit or to control the hyperalgesia and tolerance caused by opioids; the further purpose of the invention is to provide a composition of matter to produce a synergistic analgesic effect for the treatment of pain by combining an elapidae neurotoxin with an opioid, thus avoid to increase the dose of an opioid and the subsequent side effect as inhibition of respiration, constipation and addiction, allowing the long term use of opioids; finally, the invention is to provide a composition of matter for treating the patient not responding to opioid (morphine) as mono therapy, but satisfying with the combination of an opioid and an elapidae neurotoxin.

We find that elapidae neurotoxin can reduce the concentration of pro-inflammatory cytokines in spinal cord tissue, and the opioids induced hyperalgesia or analgesic tolerance is related to nerve inflammation, according to published studies, pro-inflammatory cytokines are associated with various types of pain, one of which is pathological neuralgia.

Neuropathic pain, pain caused by artificial subcutaneous formalin injection or subarachnoid injection increases significantly the secretion of IL-1B level by spinal glial cells, whilst blocking IL-1B receptors can reduce pain.[4] IL-6 can induce mechanical pain sensitivity and hyperalgesia, knockout IL-6 gene can inhibit pain in rats with sciatic nerve ligation. [5] Pro-inflammatory cytokines can increase pain in several ways, in the presence of a cytokine receptor on the neurons, pro-inflammatory cytokines may act directly on the neurons of the central nervous system to augment pain; pro-inflammatory cytokines can augment pain by modulating the transmission of incoming neural signals onto primary nerve fibers as well.

Pro-Inflammatory cytokines can also induce astrocytes and small glial cells to increase the synthesis and release of nitric oxide (NO) and activate nitric oxide synthase (NOS). These substances indirectly increase the magnitude of pain.[6-10] According to published experiment, morphine-induced hyperalgesia and tolerance are accompanied by high levels of IL-1, IL-6, NOS activity, and NO content. [11-12] In our study, we find elapidae neurotoxin can reduce these pro-inflammatory factors and relevant substances.

Studies also show that numerous nicotinic acetylcholine receptor (nAChR) acts as an important intermediate in regulating pro-inflammatory cytokines, NOS activity, and NO content, nAChR antagonists either directly reduce pro-inflammatory cytokines, NOS activity or NO content, or activate certain specific nAChR (e.g., a7-nAChR, a9-nAChR ) to reduce pro-inflammatory cytokines, NOS activity or NO content.[13-17] Other experimental results demonstrate that nAChR antagonists are directly involved in the process of reducing neuropathic pain. [18-23]

Elapidae postsynaptic neurotoxin, as the major antagonist of nicotinic acetylcholine receptor, has been shown in our experiments to be able to reduce pro-inflammatory cytokines, NOS activity and NO content, which is in line with the reported studies results of other nicotinic acetylcholine receptor antagonists for the same function.

Elapidae postsynaptic neurotoxin can antagonize acetylcholinesterase and bind to nicotinic acetylcholine receptor in a reversible manner,[24-25] structurally, they have a common three finger structure, so elapidae postsynaptic neurotoxins are also called three finger toxins, its active site is close to the end of the middle finger,[26] this three finger structure is a multifunctional structure that can regulate acetylcholine and its receptors.[27] Acetylcholine and its receptors are the main participants in cholinergic anti-inflammatory pathway, elapidae postsynaptic neurotoxin reduces the pro-inflammatory cytokines and the related substances by regulating the main participants in cholinergic anti-inflammatory pathway, this can attributes to the common property of functional structure of elapidae postsynaptic neurotoxin.

Various elapidae neurotoxin polypeptide compounds have been applied to the test in our discovery, including Asian cobra neurotoxin and king cobra neurotoxin, they have reached the same results in reducing hyperalgesia, tolerance, and in synergizing morphine's analgesic effect, which confirmed that these are the common properties of elapidae postsynaptic neurotoxin, the amino acid sequences (FASTA) of the mature proteins or peptides of these postsynaptic elapidae neurotoxins are as follows:
Postsynaptic neurotoxins of naja atra
   SEQ ID No.1
      lechnqqssq tptttgcsgg etncykkrwr dhrgyrterg cgcpsvkngi einccttdrc nn
   SEQ ID No.2
      mktllltllv vtivcldlgy tlechnqqss qtptttgcsg getncykkrw rdhrgyrter gcgcpsvkng ieinccttdr cnn
Postsynaptic neurotoxins of Bungarus multicinctus
   SEQ ID No.3
      ivchttatsp isavtcppge nlcyrkmwcd afcssrgkvv elgcaatcps kkpyeevtcc stdkcnphpk qrpg
   SEQ ID No.4
      ivchttatip ssavtcppge nlcyrkmwcd afcssrgkvv elgcaatcps kkpyeevtcc stdkcnhppk rqpg
   SEQ ID No.5
      mqcktcsfy tcpnsetcpd gknicvkrsw tavagdglkr eirrecaatc ppsklgltvf ccttdncnh
   SEQ ID No.6
      mqcktcsfy tcpnsetcpd gknicvkrsw tavrgdgpkr eirrecaatc ppsklgltvf ccttdncnh
Postsynaptic neurotoxins of Ophiophagus Hannah
   SEQ ID No.7
      ircfitpdvt sqacpdgqni cytktwcdnf cgmrgkrvdl gcaatcptvk pgvdikccst dncnpfptre rs
   SEQ ID No.8
      ircfitpdvt sqacpdghvc ytkmwcdnfc gmrgkrvdlg caatcpkvkp gvnikccsrd ncnpfptrkr s
   SEQ ID No.9
      tkcyktgdri iseacppgqd Icymktwcdv fcgtrgrvie Igctatcptv kpheqitccs tdncdphhkm Iq
Postsynaptic neurotoxins of Naja kaouthia
   SEQ ID No.10
      lechnqqssq apttktcsge tncykkwwsd hrgtiiergc gcpkvkpgvn Inccrtdrcn n
   SEQ ID No.11
      lechnqqsiq tptttgcsgg etncykkrwr dhrgyrterg cgcpsvkngi einccttdrc nn
   SEQ ID No.12

Since of our discovery is based on single compound molecule of elapidae neurotoxin which is characterized by its unique amino acid sequence, so it can be produced through recombinant technology as well, thus solves the problem of scarcity of snake venom resources; in parallel, if we continue to process these neurotoxins through isolation and purification of natural snake venom, it can allow us to control the quality and purity thanks to the amino acid sequence, this is a necessary basis for the further development to a potential drug. Finally, the application of elapidae neurotoxin single compound molecule can avoid the synergistic toxicity caused by general snake venom mixture and improve the safety of the product.

### METHOD OF IMPLEMENTATION

By establishing a mice model of morphine induced hyperalgesia and tolerance, we systematically test the inhibitory effect, synergistic effect and the ability of prolonging the analgesic time of morphine with elapidae neurotoxin polypeptide, and verify the above mentioned aspects through the model.

The following examples are provided to illustrate, but not to limit the invention.

### EXAMPLES

### Example 1. Measurement of mice's baseline pain threshold

Detailed steps were as follows:
100 Kunming mice were subjected to tail pressure tests from day 1 to day 4 days to measure the mechanical pain threshold and the average pain threshold will be set as the baseline pain threshold.
1. Put the mouse gently into the restraint and put its tail under the conical tip of the pain test apparatus. Press the pedal switch, and increase the pressure on the proximal end of the tail evenly until signs of the first pain response (struggle, squeaking) occurs. Record pain-inducing pressure when pain response occurs (units: g) as the value of pain threshold. The pressure was released upon reaching 700 g with no indication of a response to avoid tissue injury. Measurements were also conducted at the middle and distal ends of the tail of the same mouse with a minimum of 30 seconds time intervals between each measurement.
2. Put the tested mice in a cage and test the next one until every mouse in the group is tested. The average of the three measurements (i.e., the proximal, mid and distal ends of each mouse tail) was used as the pain threshold (g) of each mouse when all mice were subjected to a tail pressure test.
3. In the following 3 days, all mice underwent repeated measurements of tail pressure pain threshold.
4. The pain threshold of the mice measured by the tail pressure test ranged from 180g to 220g. The mice were then randomly divided into four groups: namely 'physiological saline group', 'morphine group', 'cobrotoxin group' and 'cobrotoxin + morphine group'. Each group comprised of 20 mice, which is used for the hyperalgesia and tolerance test of the corresponding compositions in the group's name. Any surplus mice were excluded.
5. Lastly, each group of 20 mice was divided randomly again into two groups, with each group comprising 10 mice, as amino acid sequence ID NO.1 (cobrotoxin), and amino acid sequence ID NO. 2 (cobrotoxin) will be used for parallel testing.

The average base line pain thresholds (mechanical tail pressure units (g)) of above each group of mice are presented in Fig-1 and Fig-2.

Figure-1 is the line chart of four days average baseline pain threshold (mechanical tail pressure units (g)) test results of mice randomly divided into 4 groups, namely 'physiological saline group', 'morphine group', 'cobrotoxin group', and 'cobrotoxin + morphine group'. Cobrotoxin of amino acid sequence ID No.1 was used for the test.

Figure-2 is the line chart of four days average baseline pain threshold (mechanical tail pressure units (g)) test results of mice randomly divided into 4 groups, namely 'physiological saline group', 'morphine group', 'cobrotoxin group', and 'cobrotoxin + morphine group'. Cobrotoxin of amino acid sequence ID No.2 was used for the test.

The average baseline pain threshold will be used as a benchmark to compare with pain threshold of four groups of mice: namely 'physiological saline group', 'morphine group', 'cobrotoxin group' and 'cobrotoxin + morphine group' in the next step.

### Example 2. Model establishment of morphine-Induced hyperalgesia and analgesic tolerance in mice

Principle of morphine induced hyperalgesia and analgesic tolerance model:
Mice were injected with morphine for 7 days to produce hyperalgesia and analgesic tolerance. After the first injection of morphine, the pain threshold of mice was measured before injection for 6 consecutive days as an index of morphine induced hyperalgesia; the 4th and 7th days, on the day after injection, the pain threshold of mice after injection of morphine was measured as well, the analgesic effect of morphine was measured and served as the analgesic tolerance index. These indexes can be compared with the indexes of mice in other3 control groups ("normal saline group", "cobra neurotoxin polypeptide group", "cobra neurotoxin polypeptide + morphine group") and the baseline pain threshold of mice to verify whether mice are experiencing hyperalgesia and analgesic tolerance after continuous morphine injection, cobrotoxin (amino acid sequence SEQ ID No. 1) and cobrotoxin (amino acid sequence SEQ ID No. 2) will be used in each of the following tests in parallel.

The specific steps were as follows:
1. In day 5, the measurement of pain threshold was performed before the injection. Each 4 groups of mice (total 8 groups) underwent the tail pressure test first and then were injected with morphine (5 mg/kg), sterile saline (NaCl 0.9% 1ml), cobrotoxin (50ug/kg), or cobrotoxin (50ug/kg) + morphine (5 mg/kg) respectively.
2. From the day 6 to the day 11, the measurement of pain threshold of 'physiological saline group', 'morphine group', 'cobrotoxin group' and 'cobrotoxin + morphine group' by tail pressure was performed before the injection (repeat step 1-2 of example 1) as the index of hyperalgesia; and then the method of day 5 was repeated, 4 groups of mice were injected with morphine (5 mg/kg), sterile saline (NaCl 0.9% 1ml), cobrotoxin (50ug/kg), or cobrotoxin (50ug/kg) + morphine (5 mg/kg) respectively.
3. In the 8th and 11^{th} day, an hour after the injection of 4 different compositions, the tail pressure test was applied again to measure the average pain threshold (1-2 of above example 1) of each group of the "morphine group", the "physiological saline group", the "cobrotoxin group", and the "cobrotoxin + morphine group" to determine the analgesic tolerance of these four compositions.

From day 5th to day 11th(total 7 days), an hour before the injection of 4 different compositions, the tail pressure test was applied to measure the average pain threshold (index of hyperalgesia, mechanical tail pressure units (g)) of 4 groups as shown in Fig-3 and Fig-4.

Figure-3 is the average pain threshold curve (mechanical tail pressure units (g)) measured during day 5 through day 11 (total of 7 days) of 4 groups of mice before administration of 4 different compositions which were "Physiological saline", "morphine", "cobrotoxin ", and "cobrotoxin + morphine" respectively. Cobrotoxin of amino acid sequence ID No.1 was used.

Figure-4 is the average pain threshold curve (mechanical tail pressure units (g)) measured during day 5 through day 11 (total of 7 days) of 4 groups of mice before administration of 4 different compositions which were "Physiological saline", "morphine", "cobrotoxin ", and"cobrotoxin + morphine" respectively. Cobrotoxin of amino acid sequence ID No.2 was used.

The ## symbols indicate a significant statistical difference between the average pain threshold of "morphine group" and the "cobrotoxin + morphine group", P<0.05.

In parallel, in the 5th, 8th, and 11th day, an hour after the injection of 4 different compositions, the tail pressure test was applied again to measure the average pain threshold (mechanical tail pressure units (g)) of each group of the "morphine group", the "physiological saline group", the "cobrotoxin group", and the "cobrotoxin + morphine group" to determine the analgesic tolerance of these four compositions as shown in Fig-5 and Fig-6.

Figure-5 is the pain threshold column chart (index of tolerance) of 4 different groups of mice, which were 'morphine', 'physiological saline', 'cobrotoxin ', and 'cobrotoxin + morphine' respectively. Cobrotoxin of amino acid sequence ID No.1 was used.

The mean pain threshold of the 'cobrotoxin + morphine group' is higher than that of 'cobrotoxin group', and 'morphine group' , the difference was statistically significant. The experimental data suggests that the cobrotoxin can inhibit morphine-induced analgesic tolerance, and cobrotoxin + morphine can produce a stronger analgesic effect than morphine or cobrotoxin alone.

Figure-6 is the pain threshold column chart (index of tolerance) of 4 different groups of mice , which were 'morphine', 'physiological saline', 'cobrotoxin ', and 'cobrotoxin + morphine' respectively. Cobrotoxin of amino acid sequence ID No.2 was used.

The mean pain threshold of the 'cobrotoxin + morphine group' is higher than that of 'cobrotoxin group', and 'morphine group' , the difference was statistically significant. The experimental data suggests that the cobrotoxin can inhibit morphine-induced analgesic tolerance, and cobrotoxin + morphine can produce a stronger analgesic effect than morphine or cobrotoxin alone.

Symbols ### mean a significant statistical difference between the average pain threshold of the "morphine group" and the "cobrotoxin + morphine group" for the day five, day eight, and day eleven, P<0.01; Symbols ### also indicate a significant statistical difference of average pain threshold within the morphine group of day five, day eight, and day eleven as well, P<0.01.

Symbols *** represent a significant statistical difference of average pain threshold between the "cobrotoxin group" and the "cobrotoxin + morphine group", P<0.01.

### Example 3. Synergistic Analgesic Effects of combining opioids with cobrotoxin

The specific steps were as follows:
Testing synergistic analgesic effect of morphine with cobrotoxin
1. 40 SD rats were randomly divided into "physiological saline group", "morphine group", "cobrotoxin group" and "cobrotoxin + morphine group" with 10 rats in each group
2. The aforementioned four groups of rats were injected with sterile saline (NaCl 0.9% 1ml), morphine (3 mg/kg), cobrotoxin (50ug/kg), and cobrotoxin (25ug/kg) + morphine (1.5 mg/kg) respectively.
3. 60 minutes after the injection, 1.5% acetic acid solution was injected to SD rats (1 ml/rat ). The average number of writhing within 1 hour of each group was recorded to measure the possible maximum analgesic effect of the four groups, namely "physiological saline group", "morphine group", "cobrotoxin group" and "cobrotoxin + morphine group"

Figure-7 is the column charts of rat writhing numbers counted within 1 hour after injecting 1 ml of 1.5% acetic to four groups of rats having been injected with sterile saline (NaCl 0.9% 1ml), morphine (3 mg/kg), cobrotoxin (50ug/kg), and cobrotoxin (25ug/kg) + morphine (1.5 mg/kg) respectively 60 minutes . Cobrotoxin of amino acid sequence ID No.1 was used.

Figure-8 is the column charts of rat writhing numbers counted within 1 hour after injecting 1 ml of 1.5% acetic acid to four groups of rats that have been injected with sterile saline (NaCl 0.9% 1ml), morphine (3 mg/kg), cobrotoxin (50ug/kg), and cobrotoxin (25ug/kg) + morphine (1.5 mg/kg) respectively 60 minutes before . Cobrotoxin of amino acid sequence ID No.2 was used.

Experiment results show the analgesic effect provided by half dose cobrotoxin (25ug/kg) + half dose morphine (1.5mg/kg) is significantly higher compared to a single full dose of morphine (3 mg/kg), or a single full dose of cobrotoxin (50ug/kg). This means that the cobrotoxin + morphine produces superior analgesic improvement rather than an additive one, indicating a synergistic analgesic effect.

### Example 4. Test of prolongation of morphine's analgesia effect by cobrotoxin

Following the test of example 3, the aforementioned four groups SD rats were injected with 1.5% acetic acid solution (1ml/rat) again 150 and 210 minutes after the initial injection of 4 different compositions respectively.

The test result indicated that SD rats of morphine group showed almost no signs of any analgesic effect after 150 minutes and 210 minutes; the SD rats of cobrotoxin group retained signs of analgesic effect but was inferior in comparison with the SD rats of cobrotoxin + morphine group with a significant statistical difference. The test results demonstrate the synergy formed when combining half a dose of cobrotoxin (25ug/kg) and half a dose of morphine (1.5 mg/kg) . The combination has a stronger analgesic effect than a single full dose of cobrotoxin or morphine, and this synergistic effect did not decline with the decrease of morphine's analgesic effect at 150 and 210 minutes, which showed a prolonged analgesic effect of morphine through combination with cobrotoxin as well.

Writhing numbers at time intervals of 60 minutes, 150 minutes, and 210 minutes of 4 groups of rat after injecting 1 ml of 1.5% acetic acid solution are as Fig-7 andFig-8, and beforehand, 4 different compositions have been injected to these 4 groups of rat 60 minutes before the injection of 1 ml of 1.5% acetic acid solution.

Figure-7 is the column charts of four groups of rat writhing numbers counted at time intervals of 60 minutes, 150 minutes, and 210 minutes after injecting 1 ml of 1.5% acetic acid solution and these rats have been injected with sterile saline (NaCl 0.9% 1ml), morphine (3 mg/kg), cobrotoxin (50ug/kg), and cobrotoxin (25ug/kg) + morphine (1.5 mg/kg) respectively 60 minutes before injection of 1 ml of 1.5% acetic acid . Cobrotoxin of amino acid sequence ID No.1 was used.

Figure-8 is the column charts of four groups of rat writhing numbers counted at time intervals of 60 minutes, 150 minutes, and 210 minutes after injecting 1 ml of 1.5% acetic acid solution and these four groups of rats have been injected with sterile saline (NaCl 0.9% 1ml), morphine (3 mg/kg), cobrotoxin (50ug/kg), and cobrotoxin (25ug/kg) + morphine (1.5 mg/kg) respectively 60 minutes before injection of 1 ml of 1.5% acetic acid .

Cobrotoxin of amino acid sequence ID No.2 was used.

In Fig-7 and Fig -8, symbols ### show a significant statistical difference in the number of writhing between "morphine group" and "cobrotoxin + morphine group" at 60, 150, and 210 minutes time intervals after injection, P<0.01; Symbols ### also indicate a significant statistical difference in the number of writhing within the morphine group between 60, 150, and 210 minutes time intervals after injection, P<0.01.

Symbols **and *** indicate significant statistical differences in the number of writhing between "cobrotoxin group" and "cobrotoxin + morphine group" at 60, 150, and 210 minutes time intervals after injection, **means P<0.05, ***means P<0.01 respectively.

### Example 5. Test of cobrotoxin's inhibitory effect on pro-inflammatory cytokines -studies of mechanism

The specific steps were as follows:
The measurement of IL-1β and IL-6, NOS activity, and NO

After completion of the aforementioned morphine tolerance and hyperalgesia tests, mice of "morphine group" and "cobrotoxin + morphine group" were set aside for 2 hours followed by anesthesia with chloral hydrate, and then dislocated.

The extraction of the lumbar spinal cord was performed quickly on a plate with ice, and then washed with icy water. The spinal tissues, weighed, then put into pre-frozen physiological saline, 4000 revolution/separation for 10 minutes, and prepared into 10% homogenate which was measured for IL-1β, IL-6, NOS activity, and NO content, the ELISA method was applied to determine the tested values of the lumbar spinal cord tissues, the release of IL-1β, IL-6, NOS and NO was detected according to the instruction in the insert, coomassie brilliant blue dye was used to determine the total protein content in the homogenate of each sample.

The levels of IL-1β, IL-6, NOS activity, and NO content detected in the "morphine group" and "cobrotoxin + morphine group" were as follows: (cobrotoxin of amino acid sequence ID NO. 1 was used for the test)

| Biomarker | Group | Value | SD | t-test |
|---|---|---|---|---|
| IL-1β pg/mg protein | Morphine Group | 16.80 | 2.39 | P<0.01 |
| | Morphine + cobrotoxin Group | 11.30 | 1.83 | |
| IL-6 pg/mg protein | Morphine Group | 19.40 | 2.12 | P<0.01 |
| | Morphine + cobrotoxin Group | 14.80 | 1.75 | |
| NOS U/mg protein | Morphine Group | 7.56 | 0.14 | P<0.01 |
| | Morphine + cobrotoxin Group | 7.15 | 0.12 | |
| NO µmol/g protein | Morphine Group | 1.61 | 0.03 | P<0.01 |
| | Morphine + cobrotoxin Group | 1.32 | 0.02 | |

The experimental data showed that the level of IL-1B, IL-6, NOS activity, and NO content of the "morphine group" were significantly higher than that of "cobrotoxin + morphine group".

Various elapidae neurotoxin polypeptides from the above "amino acid sequence listing" have been applied to the test and they realize the same result, which proves that these are the common properties of elapidae neurotoxin in inhibiting morphine induced hyperalgesia and tolerance, moreover, elapidae neurotoxin enhances morphine's clinical application by creating a synergistic effect with it.

Although the invention herein has been described with reference to particular embodiments, it is to be understood that these embodiments are merely illustrative of the principles and applications of the present invention. It is, therefore, to be understood that numerous modifications may be made to the illustrative embodiments and that other arrangements may be devised without departing from the spirit and scope of the present invention as defined by the appended claims.

References:
1. Naguib M et al. Advances in neurobiology of the neuromuscular junction: implications for the anesthesiologist. J Am Soc Anesthesiol, 2002, 96:202-31.
2. Abbas M, Rahman S. Effects of αα-7 nicotinic acetylcholine receptor positive allosteric modulator on lipopolysaccharide-induced neuroinflammatory pain in mice. Eur J Pharmacol, 2016, 783: 85-91.
3. Li Jiangbing et al; Research progress on the interaction between α- neurotoxin and nicotinic acetylcholine receptor. life Sciences, January 2017, Vol. 29, No. 1
4. Milligan et al. Intrathecal HIV-1 envelop glycoprotein gp 120 enhanced states mediated by spinal code proinflammatory cytokins. J neuroscience 2001(8)2808-2819.
5. Murphy et al. Endogenous interleukin-6 contribute to hypersensitivity to cutanous stimuli and changes in neuropeptides associated with chroni nerve constriction in mice.Eur J neurosci 1999,11 (7),2243-2253.
6. Xiang Hongbing et al; Effect of ketamine on spinal cord astrocytes in morphine tolerance mice. Chinese Journal of Anesthesiology, 2004,24 (4) 290-293
7. Rainer Viktor et al. The role of spinal neuroimmune activation in morphine induced tolerance/hyperplasia in neuropathic and sham operated rats. J Neurosci 2002,22(22)9980-9989.
8. Haberberger et al. Nicotinic receptor alpha7-subunits are coupled to the stimulation of nitric oxide synthase in rat dorsal root ganglion neurons∘ Histochem Cell Biol (2003) 120:173-181.
9. S. Papadopolou et al. Nicotinic receptor mediated stimulation of NO-generation in neurons of rat thoracic dorsal root ganglia. Neuroscience Letters 361 (2004) 32-35.
10. Watkins et al. Spinal cord glia new player in pain, Pain 2001,93(3):201-205.
11. Liang Huichun; Pharmacodynamic evaluation and mechanism of Guiyuan against morphine analgesia tolerance and hyperalgesia. PLA Academy of Military Sciences, 2014
12. Jian Daolin et al; Effects of morphine tolerance on IL-1 B and IL-6 levels in rat spinal cord, Journal of practical medicine, 2005 Volume 21, No. 20
13. Zakrzewicz A, J et al. (2017) Canonical and novel noncanonical cholinergic agonists inhibit ATP-induced release of monocytic interleukin-1 beta via different combinations of nicotinic acetylcholine receptor subunits alpha7, alpha9 and alpha10. Front Cell Neurosci 11, 189.
14. Patel et al. Anti-inflammatory effects of astroglial α7 nicotinic acetylcholine receptors are mediated by inhibition of the NF-κB pathway and activation of the Nrf2 pathway Journal of Neuroinflammation (2017) 14:192.
15. Papadopolou S,et al. Nicotinic receptor mediated stimulation of NO-generation in neurons of rat thoracic dorsal root ganglia. Neurosci Lett 361, 32- 35. 77 (2004).
16. Thippeswamy T,et al. Inhibition of neuronal nitric oxide synthase results in neurodegenerative changes in the axotomised dorsal root ganglion neurons: evidence for a neuroprotective role of nitric oxide in vivo. Neuroscience Research 2001.05.
17. Richter K, et al. (2016) Phosphocholine - an agonist of metabotropic but not of ionotropic functions of alpha9-containing nicotinic acetylcholine receptors. Sci Rep 6, 28660.
18. Pacini A et al. The alpha9alpha10 nicotinic receptor antagonist alpha-conotoxin RgIA prevents neuropathic pain induced by oxaliplatin treatment. Exp Neurol 2016.282, 37-48.
19. Romero HK et al. Inhibition of a9a10 nicotinic acetylcholine receptors prevents chemotherapy-induced neuropathic pain. Proc Natl Acad Sci USA 2017.114, 1825-1832.
20. Vincler M et al. Molecular mechanism for analgesia involving specific antagonism of alpha9alpha10 nicotinic acetylcholine receptors. Proc Natl Acad Sci USA 2006.103,17880-17884.
21. Luo S,et al. Cloning, synthesis, and characterization of alphaOconotoxin GeXIVA, a potent alpha9alpha10 nicotinic acetylcholine receptor antagonist. Proc Natl Acad Sci USA 2015.112, E4026-E4035.
22. Holtman JR et al. The novel small molecule alpha9alpha10 nicotinic acetylcholine receptor antagonist ZZ-204G is analgesic. Eur J Pharmacol 2011.670, 500-508.
23. Wala EP et al. Novel small molecule alpha9alpha10 nicotinic receptor antagonist prevents and reverses chemotherapy-evoked neuropathic pain in rats. Anesth Analg . 2012.115, 713-720.
24. Naguib M et al. Advances in neurobiology of the neuromuscular junction: implications for the anesthesiologist. J Am Soc Anesthesiol. 96:202-31, 2002.
25. Abbas M, Rahman S. Effects of α-7 nicotinic acetylcholine receptor positive allosteric modulator on lipopolysaccharide-induced neuroinflammatory pain in mice. Eur J Pharmacol. 783: 85-91, 2016.
26. J. White et al, 1996 Snake Neurotoxin. Human Toxicology, 1996.
27. Pascale Marchot et al, The three-finger toxin fold: a multifunctional structural scaffold able to modulate cholinergic functions Journal of neurochemistry, Vol 142, issue S2, 2017.

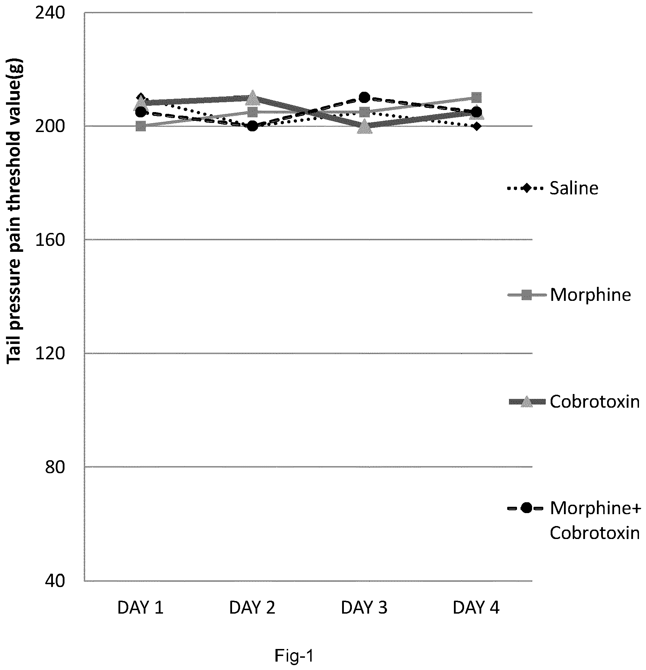

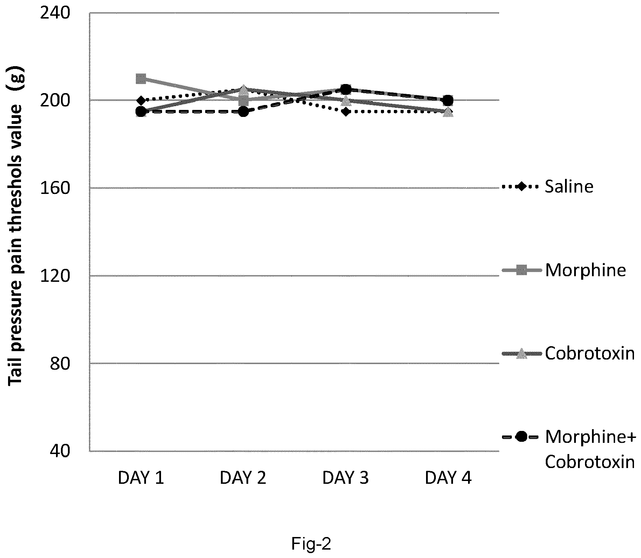

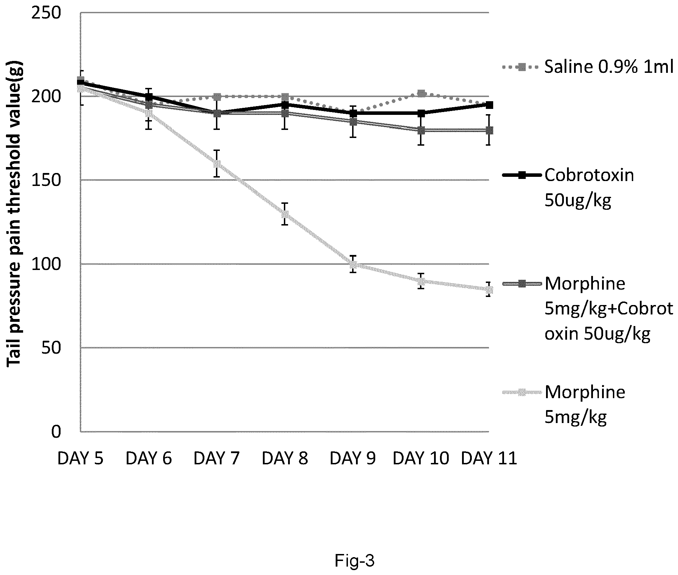

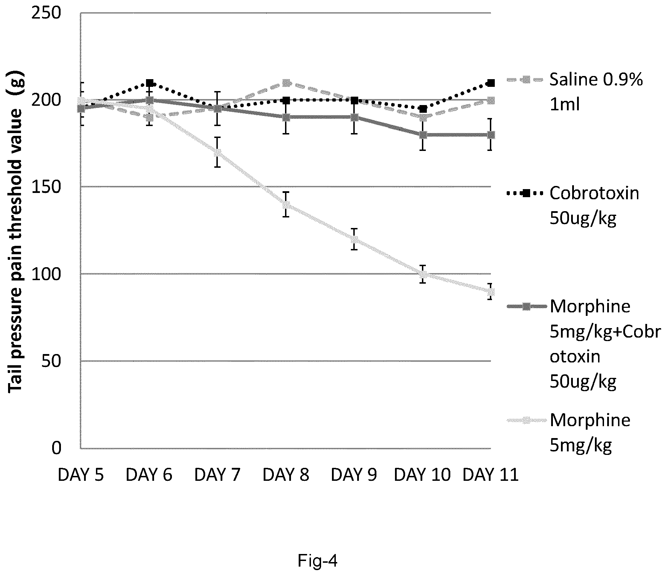

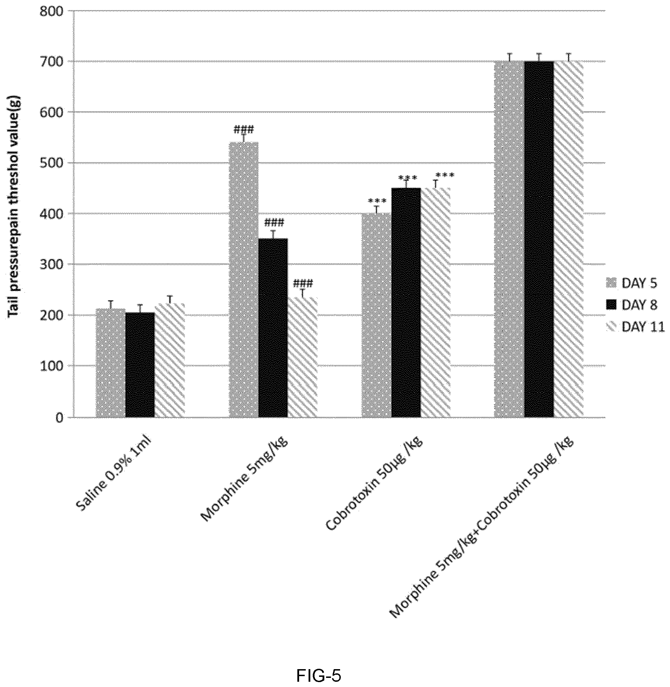

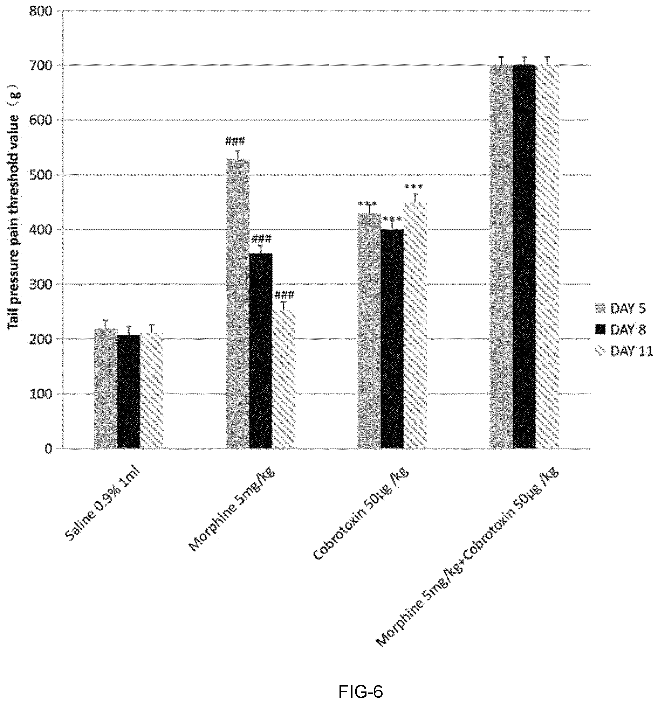

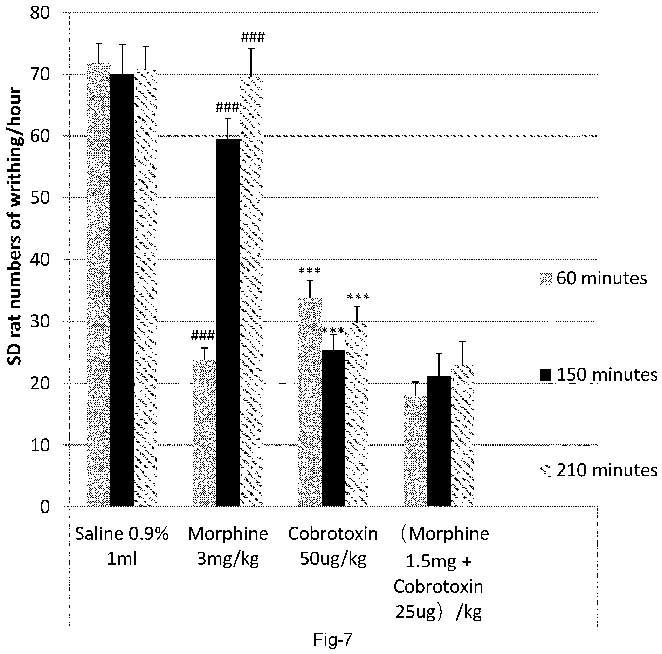

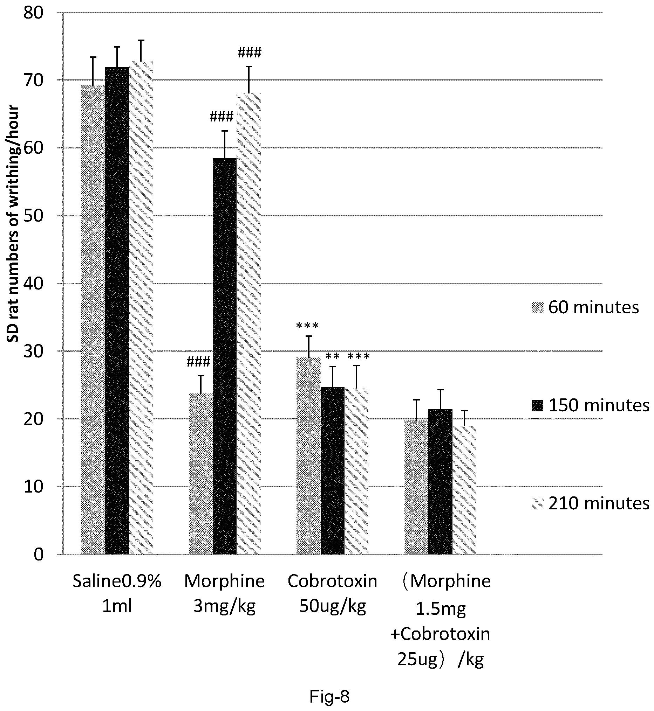

## Claims

1. A method for the treatment of opioids induced hyperalgesia in a mammal, wherein said method comprising administering to a mammal in need thereof a pharmaceutical composition of a therapeutically effective amount of elapidae neurotoxin, and a pharmaceutically acceptable carrier base for use in inhibiting or controlling opioids induced hyperalgesia.

2. A method for the treatment of opioids induced tolerance in a mammal, wherein said method comprising administering to a mammal in need thereof a pharmaceutical composition of a therapeutically effective amount of elapidae neurotoxin, and a pharmaceutically acceptable carrier base for use in inhibiting or controlling opioids induced tolerance.

3. A method for the treatment of pain in a mammal, wherein said method comprising administering to a mammal in need thereof a pharmaceutical composition of a therapeutically effective amount of elapidae neurotoxin, in combination with an opioid drug, and a pharmaceutically acceptable carrier base for use in producing synergistic analgesic effect for the patients not responding to an opioid drug as analgesia.

4. A method for the treatment of pain in a mammal, wherein said method comprising administering to a mammal in need thereof a pharmaceutical composition of a therapeutically effective amount of elapidae neurotoxin, and a pharmaceutically acceptable carrier base for use in prolonging the analgesic effect of an opioid drug for the treatment of pain .

5. The method of any one or more of claims 1 to 4, wherein said elapidae neurotoxin is **characterized in that** it is a elapidae neurotoxin polypeptide having the amino acid sequence shown in SEQ ID No. 1 to SEQ ID No. 12; or elapidae neurotoxin polypeptide homologues having 70% or more homology with the elapidae neurotoxin polypeptide of SEQ ID No. 1 to SEQ ID No. 12, and the biological function of the elapidae neurotoxin polypeptide homologues is the same as or similar to that of the elapidae neurotoxin polypeptide of the amino acid sequence ID No. 1 to SEQ ID No. 12.

6. The method of any one or more of claims 1 to 5, wherein said elapidae neurotoxin polypeptides or elapidae neurotoxin polypeptides homologues is selected from the isolation of natural snake venoms, or from synthesized chemical polypeptides, or from prokaryotic or eukaryotic hosts using recombinant technology (such as Bacteria, yeast, higher plants, insects and mammalian cells).

7. The method of any one or more of claims 1 to 6, wherein said recombinant technology produced elapidae neurotoxin polypeptide or its homologues, based on the host used in the recombinant production scheme, the polypeptide or its homologues of the present invention may be glycosylated, or may be non-glycosylated; Disulfide-bonded or non-disulfide-bonded. The polypeptides and its homologues described in the present invention may also include or exclude the starting methionine residue.

8. The method of any one or more of claims 1 to 7, wherein said elapidae neurotoxin polypeptide is further **characterized in that** the polypeptide in the present invention may include fragments of the above-mentioned elapidae neurotoxin polypeptides after hydrolysis or enzymolysis, derivatives or analogs treated by physical, chemical or biological method, they are polypeptides which basically maintain the same biological function or activity as the above-mentioned elapidae neurotoxin polypeptide. The fragments, derivatives or analogs described in the present invention may be a polypeptide in which one or more amino acid residues are substituted, or a polypeptide having a substituent group in one or more amino acid residues, or combined with a compound (such as compounds that extend the half-life of a polypeptide, such as polyethylene glycol), or a polypeptide formed by fusion of a fatty chain, or a polypeptide formed by fusing an additional amino acid sequence to this polypeptide sequence. As described herein, these fragments, derivatives, and analogs are within the scope of those skilled in the art.

9. The method of any one or more of claims 1 to 4, wherein said pain is acute or chronic pain, including traumatic pain, somatic pain, visceral pain, neuropathic pain, post-operative pain, cancer pain, inflammatory pain, fibromyalgia, toothache, Dysmenorrhea, kidney pain, headache, biliary colic, arthralgia, back pain, arthroscopic pain, gynecological laparoscopic pain, and pain caused by burns, rheumatoid arthritis, and intraocular hypertension.

10. The method of any one or more of claims 1 to 4, wherein the administration comprising intravenous, intramuscular, subcutaneous, intra-articular, oral, sublingual, nasal, rectal, topical, intradermal, intraperitoneal, intrathecal administration or transdermal administration; the dose of elapidae neurotoxin includes from 1 µg / Kg to 350 µg / kg each time, and the injection frequency ranges from once a day to multiple times a day.
